# FASCICULE DE BREVET EUROPEEN

(11) **EP 3 938 356 B1**
(45) Date de publication et mention de la délivrance du brevet: **18.09.2024**
(21) Numéro de dépôt: 20725864.1
(22) Date de dépôt: 10.03.2020
(51) Int. Cl.: C07D 265/16, C07D 407/06, C08G 73/02, C08G 14/06

(54) **PROCEDE DE FABRICATION D'UN MONOMERE POLYBENZOXAZINE**
VERFAHREN ZUR HERSTELLUNG EINES POLYBENZOXAZIN-MONOMERS
PROCESS FOR THE PRODUCTION OF A POLYBENZOXAZINE MONOMER

(30) Priorité: 15.03.2019 FR 1902694
(43) Date de publication de la demande: 19.01.2022
(73) Titulaire: ArianeGroup SAS, 78130 Les Mureaux (FR); Centre national de la recherche scientifique, 75016 Paris (FR); UNIVERSITE DE MONTPELLIER, 34090 Montpellier (FR); Ecole Nationale Supérieure de Chimie de Montpellier, 34296 Montpellier (FR)
(72) Inventeur: TAVERNIER, Romain, 80600 DOULLENS (FR); GRANADO, Lérys, 11000 CARCASSONNE (FR); DAVID, Ghislain, 34080 MONTPELLIER (FR); CAILLOL, Sylvain, 34090 MONTPELLIER (FR); FOYER, Gabriel, 33700 MERIGNAC (FR)
(74) Mandataire: Cabinet Beau de Loménie
(86) Numéro de dépôt international: PCT/FR2020/050484
(87) Numéro de publication internationale: WO 2020/188182

(56) Documents cités:
- WO-A1-01/34581
- WO-A1-92/16470
- US-A1- 2017 158 877
- HAJIME KANATOMI ET AL: "Reaction of Salicylamine with [alpha]-Dicarbonyl Compounds. II. Formation of 2,2'-Bibenz-1,3-oxazines", BULLETIN OF THE CHEMICAL SOCIETY OF JAPAN, vol. 43, no. 1, January 1970 (1970-01-01), JP, pages 226 - 231, XP055626769, ISSN: 0009-2673, DOI: 10.1246/bcsj.43.226
- SEISHI OHASHI ET AL: "Synthesis and ring-opening polymerization of 2-substituted 1,3-benzoxazine: the first observation of the polymerization of oxazine ring-substituted benzoxazines", POLYMER CHEMISTRY, vol. 7, no. 46, November 2016 (2016-11-01), pages 7177 - 7184, XP055626808, ISSN: 1759-9954, DOI: 10.1039/C6PY01686C
- ZILONG TANG ET AL: "Efficient Synthesis of 2,3-Disubstituted-1,3-benzoxazines by Chlorotrimethylsilane-Mediated Aza-Acetalizations of Aromatic Aldehydes : TMSCl-Catalyzed Synthesis of 2,3-Disubstituted-1,3-benzoxazines", JOURNAL OF HETEROCYCLIC CHEMISTRY, August 2013 (2013-08-01), US, pages n/a - n/a, XP055626764, ISSN: 0022-152X, DOI: 10.1002/jhet.1590
- ZI-LONG TANG ET AL: "Synthesis and fungicidal activity of novel 2-aryl-3-(1,3,4-thiadiazolyl)-6(8)-methyl-1,3-benzoxazines", BIOORGANIC & MEDICINAL CHEMISTRY LETTERS, vol. 25, no. 16, 15 May 2015 (2015-05-15), AMSTERDAM, NL, pages 3378 - 3381, XP055626759, ISSN: 0960-894X, DOI: 10.1016/j.bmcl.2015.05.010

## Description

### Domaine Technique

L'invention concerne un procédé de fabrication d'un monomère polybenzoxazine et la réticulation de ce monomère afin de former un produit réticulé ayant des propriétés de charbonnement et de stabilité thermique améliorées. Ce produit réticulé peut notamment être utilisé en tant que résine ablative pour la constitution d'une tuyère de propulseur ou d'un corps de rentrée atmosphérique.

### Technique antérieure

Il est connu de réaliser des protections thermiques ablatives telles que celles des tuyères de propulseurs ou des corps de rentrée atmosphérique à partir de résines phénoliques synthétisées à partir de formaldéhyde et de phénol, afin d'obtenir des densités d'aromatiques et de réticulation élevées, et donc un taux de coke important. Le formaldéhyde et le phénol sont toutefois des composés classés Cancérigène Mutagène Reprotoxique (CMR) de catégorie 1B et 2 dont il serait souhaitable de limiter l'usage, notamment pour anticiper d'éventuelles interdictions en Union Européenne. En outre, la réaction du phénol et du formaldéhyde est une polycondensation pendant laquelle de l'eau est produite. Cette eau peut se retrouver piégée au sein du produit fini, conduisant à une baisse des performances. Dans l'optique de résoudre ce problème, des développements ont été poursuivis afin d'obtenir des résines ablatives par réticulation de benzoxazines. Ces développements réduisent le piégeage de l'eau dans le produit fini mais les solutions de la littérature, fournissant un produit fini avec des propriétés de stabilité thermique et de charbonnement compatibles d'une application en tant que résine ablative, mettent en jeu des benzoxazines obtenues par réaction du phénol avec le formaldéhyde et une amine, et se basent donc sur la mise en oeuvre de composés classés CMR. De plus, lorsque ces benzoxazines sont synthétisées sans phénol, elles présentent des propriétés de stabilité thermique et de charbonnement peu performantes.

On connaît la publication HAJIME KANATOMI ET AL: "Reaction of Salicylamine with Dicarbonyl Compounds. II. Formation of 2,2'-Bibenz-1,3-oxazines", BULLETIN OF THE CHEMICAL SOCIETY OF JAPAN, vol. 43, no. 1, janvier 1970 (1970-01), pages 226-231, qui divulgue une synthèse par réaction de salicylamines avec du glyoxal dans le méthanol à une température inférieure à 20°C. On connaît également US 2017/158877 qui divulgue l'utilisation de benzoxazines dans un système de protection thermique, WO 92/16470 qui divulgue la formation de composites carbone-carbone utilisant des benzoxazines en tant que précurseur de carbone, et WO 01/34581 qui divulgue des compositions à base de benzoxazines à haut taux de charbonnement.

Il serait par conséquent souhaitable de disposer d'une nouvelle voie de synthèse de benzoxazines limitant l'emploi de composés CMR, et s'affranchissant en particulier de l'utilisation de formaldéhyde.

Il serait en outre souhaitable de disposer d'une nouvelle voie de synthèse de matériaux présentant des propriétés de stabilité thermique et de charbonnement adaptées à la réalisation de protections thermiques ablatives pour tuyères de propulseurs et corps de rentrée atmosphérique.

### Exposé de l'invention

L'invention concerne un procédé de fabrication d'un monomère polybenzoxazine de formule C selon les revendications, qui comprend la réaction d'une amine de formule A avec un polyaldéhyde de formule B; les formules A, B et C étant comme définies dans la revendication 1.

L'invention fournit une nouvelle voie de synthèse de polybenzoxazines limitant l'utilisation de composés CMR, et s'affranchissant en particulier de l'emploi de formaldéhyde. En outre, le produit obtenu par réticulation du monomère polybenzoxazine de formule C ci-dessus présente de hautes propriétés de stabilité thermique et de charbonnement, compatibles d'une application en tant que résine ablative pour tuyère de propulseur ou corps de rentrée atmosphérique.

R₁^{a} est est un groupe furfuryle substitué ou non substitué ou un groupe benzyle substitué ou non substitué.

Le choix de tels groupements R₁^{a} permet avantageusement d'obtenir après réticulation du monomère polybenzoxazine de formule C un produit présentant des propriétés de stabilité thermique et de charbonnement améliorées.

En particulier, R₁^{a} peut être un groupe furfuryle substitué ou non substitué.

La présence de l'oxygène du cycle furfuryle permet, en acceptant les liaisons hydrogène, d'apporter une stabilisation accrue du réseau réticulé, et d'augmenter ainsi davantage encore le taux de charbonnement.

Dans un exemple de réalisation, n^{a} est égal à 0.

Dans la variante où n^{a} est non nul, R₂^{a} est choisi parmi : les groupes alcoxy, les groupes carboxyles, les atomes d'halogène, les chaînes hydrocarbonées linéaires ou ramifiées comprenant entre 1 et 6 atomes de carbone, saturées ou insaturées, substituées ou non substituées, interrompues ou non par un ou plusieurs hétéroatomes, les groupes carbocycliques ou hétérocycliques saturés, insaturés ou aromatiques, substitués ou non substitués.

L'amine de formule A peut être une monoamine, c'est-à-dire ne comprendre qu'une seule fonction amine.

Dans un exemple de réalisation, le procédé peut comprendre, avant la condensation, l'obtention de l'amine de formule A, cette obtention comprenant :
- une réaction d'addition d'une amine de formule A1 sur un aldéhyde de formule A2 afin de former une imine de formule A3, et
- une réaction de réduction de l'imine de formule A3 en amine de formule A,
les formules A1, A2 et A3 étant fournies ci-dessous:

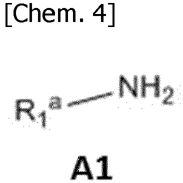

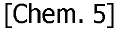

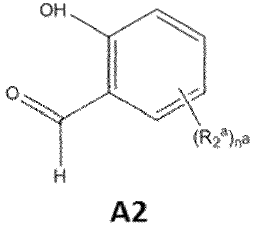

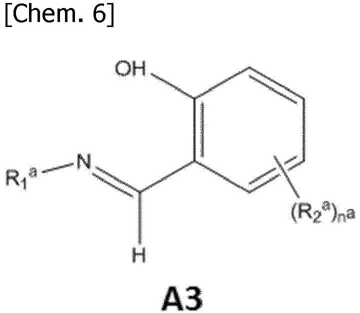

L'amine de formule A1 est par exemple choisie parmi les composés suivants : la furfurylamine, ou la benzylamine.

L'aldéhyde de formule A2 peut par exemple être le salicylaldehyde.

B₁ est choisi parmi les groupes carbocycliques aromatiques ou hétérocycliques aromatiques, monocycliques ou polycycliques, substitués ou non substitués.

Le choix d'un tel polyaldéhyde permet avantageusement d'obtenir après réticulation du monomère polybenzoxazine de formule C un produit présentant des propriétés de stabilité thermique et de charbonnement améliorées.

En particulier, B₁ peut être un cycle benzénique substitué ou non substitué et dans ce cas N₁ = 2 ou 3.

Le polyaldéhyde de formule B est par exemple choisi parmi les composés suivants : téréphthalaldéhyde, isophthalaldéhyde.

D'une manière générale et quel que soit l'exemple de réalisation considéré, N₁ peut être un entier égal à 2. Dans ce cas, le polyaldéhyde de formule B est un dialdéhyde.

N₁ peut en variante prendre d'autres valeurs, N₁ peut en particulier être égal à 3 ou plus.

Quel que soit le mode de réalisation considéré, la condensation peut être réalisée en portant le mélange de l'amine de formule A avec le polyaldéhyde de formule B au reflux. On peut réaliser la condensation dans le toluène, le méthanol, l'éthanol ou encore sans solvant.

L'invention concerne également un procédé de fabrication d'un produit réticulé comprenant la réticulation du monomère polybenzoxazine de formule C obtenu par mise en oeuvre du procédé tel que décrit plus haut.

Ce produit réticulé présente un haut taux de coke et une haute stabilité thermique, qui le rendent compatible d'une application en tant que résine ablative pour la fabrication d'une tuyère de propulseur ou d'un corps de rentrée atmosphérique.

La réticulation peut être effectuée en imposant une température supérieure ou égale à 130°C, par exemple comprise entre 180°C et 250°C.

Dans un exemple de réalisation, il y a réticulation d'un mélange comprenant le monomère polybenzoxazine de formule C et un monomère monobenzoxazine additionnel de formule D, la formule D étant fournie ci-dessous : 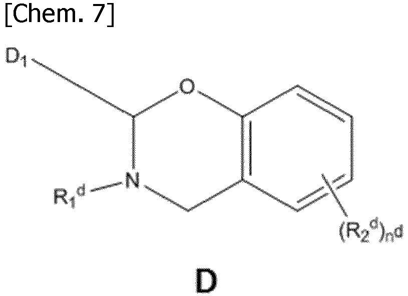 formule dans laquelle :
R₁^{d} est choisi parmi : les groupes furfuryles, substitués ou non substitués, les groupes carbocycliques ou hétérocycliques saturés, insaturés ou aromatiques, monocycliques ou polycycliques, substitués ou non substitués, les groupes aralkyles substitués ou non substitués, les chaînes hydrocarbonées linéaires ou ramifiées, saturées ou insaturées, substituées ou non substituées, interrompues ou non par un ou plusieurs hétéroatomes ou par un ou plusieurs groupes carbocycliques ou hétérocycliques saturés, insaturés ou aromatiques, monocycliques ou polycycliques, substitués ou non substitués,
R₂^{d} est choisi parmi : les groupes alcoxy, les groupes carboxyles, les atomes d'halogène, les chaînes hydrocarbonées linéaires ou ramifiées comprenant entre 1 et 6 atomes de carbone, saturées ou insaturées, substituées ou non substituées, interrompues ou non par un ou plusieurs hétéroatomes, les groupes carbocycliques ou hétérocycliques saturés, insaturés ou aromatiques, substitués ou non substitués, n^{d} est un entier compris entre 0 et 2,
D₁ est choisi parmi : les groupes carbocycliques ou hétérocycliques saturés, insaturés ou aromatiques, monocycliques ou polycycliques, substitués ou non substitués, les chaînes hydrocarbonées linéaires ou ramifiées, saturées ou insaturées, substituées ou non substituées, interrompues ou non par un ou plusieurs hétéroatomes,
et, lorsqu'ils sont substitués, chacun de R₁^{d}, R₂^{d} et D₁ étant substitué par l'un au moins des groupements suivants : hydroxyméthyle, méthyle, acide carboxylique.

Le fait d'adjoindre le monomère monobenzoxazine additionnel de formule D permet de faciliter l'obtention d'un milieu liquide comprenant le monomère polybenzoxazine de formule C à température modérée et sans avoir recours à un solvant aromatique ou halogéné. Cela facilite l'utilisation du mélange dans des procédés comme le moulage par transfert de résine (« Resin Transfer Molding » ; « RTM ») sans significativement affecter les propriétés thermiques et de charbonnement du produit réticulé obtenu.

On peut par exemple réticuler un mélange comprenant le monomère polybenzoxazine de formule C à raison de 20% à 80% en masse et de préférence de 20% à 50% en masse, et le monomère monobenzoxazine additionnel de formule D à raison de 20% à 80% en masse et de préférence de 50% à 80% en masse.

Dans un exemple de réalisation, D₁ est choisi parmi les groupes carbocycliques aromatiques ou hétérocycliques aromatiques, monocycliques ou polycycliques, substitués ou non substitués.

En particulier, D₁ peut être un cycle benzénique substitué ou non substitué.

D₁ peut en variante être une chaîne hydrocarbonée linéaire ou ramifiée. Le nombre d'atomes de carbone de cette chaîne hydrocarbonée peut varier dans de larges proportions, D₁ peut comprendre entre 1 et 20 atomes de carbone ou être un polymère.

R₁^{d} peut en particulier être choisi parmi : les groupes furfuryles, substitués ou non substitués, les groupes carbocycliques aromatiques ou hétérocycliques aromatiques, monocycliques ou polycycliques, substitués ou non substitués, les groupes aralkyles substitués ou non substitués. En particulier, R₁^{d} peut être un groupe furfuryle substitué ou non substitué. R₁^{d} est en variante une chaîne hydrocarbonée linéaire ou ramifiée. Le nombre d'atomes de carbone de cette chaîne hydrocarbonée peut varier dans de larges proportions. R₁^{d} peut par exemple être une chaîne hydrocarbonée linéaire ou ramifiée comprenant entre 1 et 20 atomes de carbone ou être un polymère.

Dans un exemple de réalisation, n^{d} est égal à 0. Dans la variante où n^{d} est non nul, R₂^{d} est choisi parmi : les groupes alcoxy, les groupes carboxyles, les atomes d'halogène, les chaînes hydrocarbonées linéaires ou ramifiées comprenant entre 1 et 6 atomes de carbone, saturées ou insaturées, substituées ou non substituées, interrompues ou non par un ou plusieurs hétéroatomes, les groupes carbocycliques ou hétérocycliques saturés, insaturés ou aromatiques, substitués ou non substitués.

Le monomère monobenzoxazine additionnel de formule D peut être obtenu par condensation entre une amine similaire à l'amine de formule A avec un monoaldéhyde de formule D₁-CHO (ne comportant qu'une seule fonction aldéhyde).

En variante, il y a réticulation d'un mélange comprenant le monomère polybenzoxazine de formule C et un monomère polybenzoxazine additionnel de formule E, la formule E étant fournie ci-dessous : 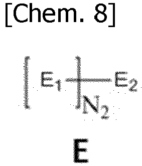 dans cette formule E :
N₂ est un entier supérieur ou égal à 2,
E₂ est choisi parmi les chaînes hydrocarbonées linéaires ou ramifiées saturées ou insaturées, substituées ou non substituées, interrompues par un ou plusieurs hétéroatomes ou par un ou plusieurs groupes carbocycliques ou hétérocycliques saturés, insaturés ou aromatiques, monocycliques ou polycycliques, substitués ou non substitués, ou non interrompues,
les groupements de formule E₁ sont identiques ou différents et présentent chacun la formule ci-dessous : 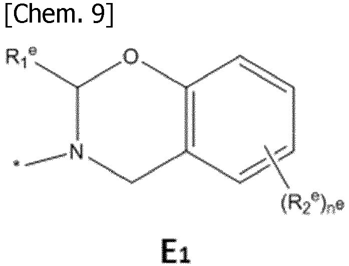 dans cette formule E₁ :
   R₁^{e} est choisi parmi : les groupes carbocycliques ou hétérocycliques saturés, insaturés ou aromatiques, monocycliques ou polycycliques, substitués ou non substitués, les chaînes hydrocarbonées linéaires ou ramifiées, saturées ou insaturées, substituées ou non substituées, interrompues ou non par un ou plusieurs hétéroatomes,
   R₂^{e} est choisi parmi : les groupes alcoxy, les groupes carboxyles, les atomes d'halogène, les chaînes hydrocarbonées linéaires ou ramifiées comprenant entre 1 et 6 atomes de carbone, saturées ou insaturées, substituées ou non substituées, interrompues ou non par un ou plusieurs hétéroatomes, les groupes carbocycliques ou hétérocycliques saturés, insaturés ou aromatiques, substitués ou non substitués, n^{e} est un entier compris entre 0 et 2,
   *- désigne la liaison à E₂
   et, lorsqu'ils sont substitués, chacun de E₂, R₁^{e} et R₂^{e} étant substitué par l'un au moins des groupements suivants : hydroxyméthyle, méthyle, acide carboxylique.

Le fait d'adjoindre le monomère polybenzoxazine additionnel de formule E permet de faciliter l'obtention d'un milieu liquide comprenant le monomère polybenzoxazine de formule C à température modérée et sans avoir recours à un solvant aromatique ou halogéné. Cela facilite l'utilisation du mélange dans des procédés comme le moulage par transfert de résine (« Resin Transfer Molding » ; « RTM ») sans significativement affecter les propriétés thermiques et de charbonnement du produit réticulé obtenu.

On peut par exemple réticuler un mélange comprenant le monomère polybenzoxazine de formule C à raison de 20% à 80% en masse, de préférence de 20% à 50% en masse, et le monomère polybenzoxazine additionnel de formule E à raison de 20% à 80% en masse, de préférence de 50% à 80% en masse.

R₁^{e} peut être choisi parmi : les groupes carbocycliques aromatiques ou hétérocycliques aromatiques, monocycliques ou polycycliques, substitués ou non substitués. En particulier, R₁^{e} peut être un cycle benzénique substitué ou non substitué.

Dans un exemple de réalisation, n^{e} est égal à 0. Dans la variante où n^{e} est non nul, R₂^{e} est choisi parmi : les groupes alcoxy, les groupes carboxyles, les atomes d'halogène, les chaînes hydrocarbonées linéaires ou ramifiées comprenant entre 1 et 6 atomes de carbone, saturées ou insaturées, substituées ou non substituées, interrompues ou non par un ou plusieurs hétéroatomes, les groupes carbocycliques ou hétérocycliques saturés, insaturés ou aromatiques, substitués ou non substitués.

E₂ peut être choisi parmi : les chaînes hydrocarbonées linéaires ou ramifiées, saturées ou insaturées, substituées ou non substituées, interrompues par un ou plusieurs groupes carbocycliques aromatiques ou hétérocycliques aromatiques. E₂ peut en particulier être un groupement xylylène substitué ou non substitué. Selon une variante, E₂ peut être choisi parmi les chaînes hydrocarbonées linéaires ou ramifiées, saturées ou insaturées, substituées ou non substituées, non interrompues.

N₂ peut être égal à 2.

De manière similaire à ce qui a été décrit plus haut, le composé de formule E peut être obtenu par condensation d'une polyamine avec un aldéhyde de formule R₁^{e}-CHO.

Quel que soit le mode de réalisation considéré, la proportion de monomère monobenzoxazine additionnel de formule D ou de monomère polybenzoxazine additionnel de formule E peut être choisie de sorte que le mélange soit liquide lorsqu'il est porté à une température comprise entre 20°C et 100°C.

Lorsqu'ils sont substitués, chacun de R₁^{a}, B₁, R₂^{a}, R₁^{d}, R₂^{d}, D₁, E₂, R₁^{e}, R₂^{e} est substitué par l'un au moins des groupements suivants : hydroxyméthyle, méthyle, acide carboxylique.

L'invention concerne également un procédé de fabrication d'une tuyère de propulseur dans lequel la tuyère est fabriquée en mettant en oeuvre un produit réticulé obtenu par le procédé décrit plus haut.

La tuyère de propulseur peut être en matériau composite. Dans ce cas, la fabrication de la tuyère peut comporter une première étape de formation d'une préforme fibreuse de la tuyère à obtenir imprégnée par le monomère polybenzoxazine de formule C obtenu comme décrit plus haut ou par un mélange comprenant ce monomère tel que décrit plus haut. Cette fabrication peut en outre comporter une deuxième étape traitement thermique de la préforme fibreuse imprégnée de manière à réticuler le monomère polybenzoxazine de formule C ou le mélange et obtenir la tuyère de propulseur.

La préforme fibreuse peut par exemple comporter des fibres de carbone, de silice, de verre ou d'un matériau céramique, par exemple de carbure de silicium. La préforme fibreuse destinée à former le renfort fibreux de la tuyère peut être formée de diverses manières (drapage de couches de tissu pré-imprégnées, par exemple). On peut ainsi en particulier draper ou bobiner des couches de tissu bi-dimensionnel ou tri-dimensionnel imprégnées sur une forme ayant une surface reproduisant la géométrie désirée d'une surface interne ou externe de la tuyère à réaliser afin d'obtenir la préforme imprégnée. En variante, on peut d'abord obtenir la préforme fibreuse de la tuyère à obtenir puis placer cette préforme dans une cavité d'injection et injecter ensuite le monomère polybenzoxazine de formule C ou le mélange comprenant ce monomère décrit plus haut dans la cavité de manière à imprégner la préforme. Dans ce cas, on peut mettre en oeuvre une technique de moulage par transfert de résine (« Resin Transfer Molding ») pour imprégner la préforme fibreuse.

L'invention concerne également un procédé de fabrication d'un corps de rentrée atmosphérique dans lequel le corps de rentrée atmosphérique est fabriqué en mettant en oeuvre un produit réticulé obtenu par le procédé décrit plus haut.

### Brève description des dessins

[Fig. 1] La figure 1 est un thermogramme DSC d'un exemple de monomère polybenzoxazine de formule C obtenu par mise en oeuvre de l'invention.
[Fig. 2] La figure 2 est un résultat d'analyse thermogravimétrique (« thermogravimetric analysis » ; « TGA ») d'un produit réticulé obtenu par réticulation de ce monomère polybenzoxazine.
[Fig. 3] La figure 3 est un thermogramme DSC d'un exemple de monomère polybenzoxazine de formule C obtenu par mise en oeuvre de l'invention.
[Fig. 4] La figure 4 est un résultat d'analyse thermogravimétrique d'un produit réticulé obtenu par réticulation de ce monomère polybenzoxazine.
[Fig. 5] La figure 5 est un thermogramme DSC d'un exemple de monomère polybenzoxazine de formule C obtenu par mise en oeuvre de l'invention.
[Fig. 6] La figure 6 est un résultat d'analyse thermogravimétrique d'un produit réticulé obtenu par réticulation de ce monomère polybenzoxazine.
[Fig. 7] La figure 7 est un thermogramme DSC d'un exemple de monomère polybenzoxazine additionnel de formule E utilisable dans le cadre du procédé selon l'invention.
[Fig. 8] La figure 8 est un résultat d'analyse thermogravimétrique d'un produit réticulé obtenu par réticulation de ce monomère polybenzoxazine.

### Description des modes de réalisation

### Exemples

### Exemple 1 : synthèse d'un monomère polybenzoxazine à partir de furfurylaminométhylphénol et téréphthalaldéhyde puis réticulation subséquente

On fait réagir la furfurylamine avec le salicylaldéhyde dans des proportions stoechiométriques dans le méthanol au reflux pendant 2h, dans le but de former l'imine correspondante. L'imine est réduite en amine avec 1 équivalent de NaBH₄ ajouté à 0°C dans une solution de l'imine dans le MeOH, puis on réalise un chauffage à reflux pendant 2 heures. Le furfurylaminométhylphénol ainsi synthétisé est dissous dans le toluène avec 0,5 équivalent de téréphthalaldéhyde, puis porté au reflux dans un appareil Dean Stark, afin de retirer l'eau générée pendant la réaction de condensation. La réaction est arrêtée lorsque la conversion des aldéhydes a atteint son maximum, suivi par RMN du proton. Après évaporation du solvant sous pression réduite, la bisbenzoxazine isolée est un solide blanc cassé. Le produit a été caractérisé par RMN et spectroscopie infrarouge et la structure a été confirmée.

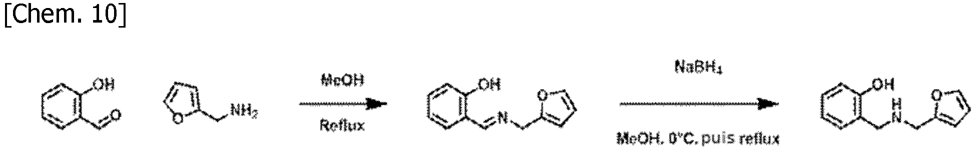

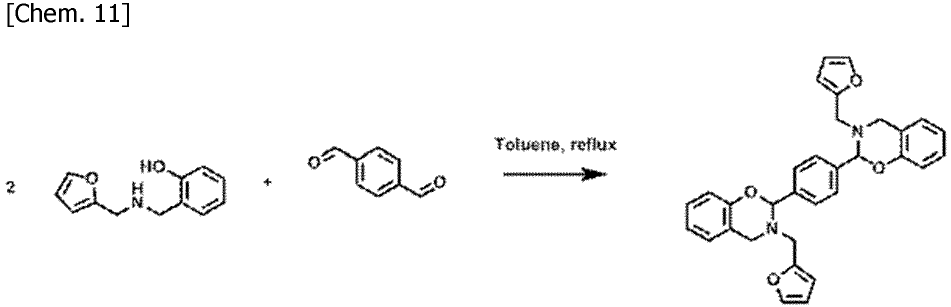

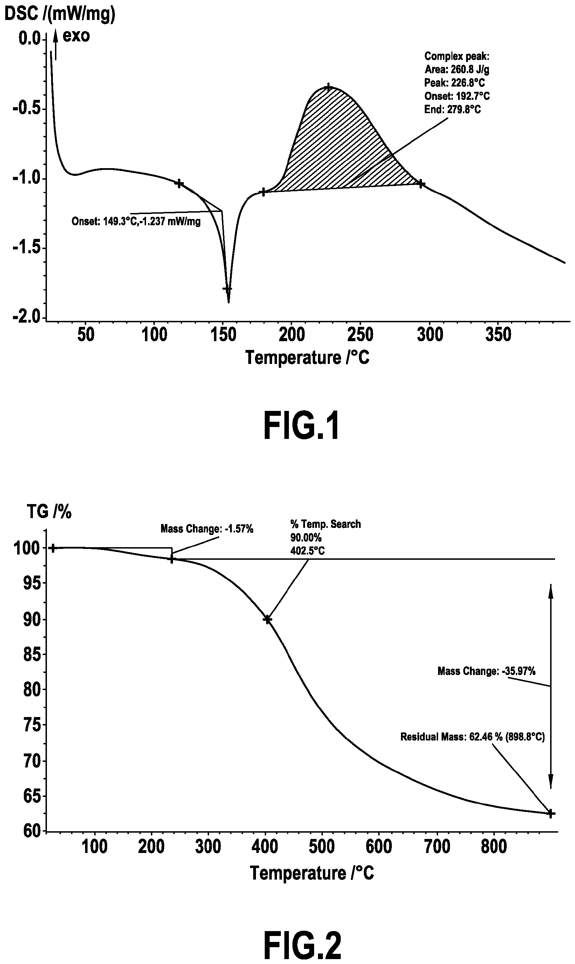

La caractérisation thermique par calorimétrie différentielle à balayage (« Differential Scanning Calorimetry » ; « DSC ») a révélé une température de fusion de 150°C ainsi qu'une réaction exothermique entre 190°C et 280°C, représentant 261J/g d'enthalpie par rapport à la référence, avec une rampe de 20°C/min dans des creusets en acier scellés haute pression. Le thermogramme DSC obtenu est fourni à la figure 1.

Un échantillon de bisbenzoxazine a été réticulé à 180°C pendant 4 heures et n'a pas montré de signal résiduel en DSC. L'analyse thermogravimétrique a montré un taux de coke de 62% sous atmosphère d'azote, après 1h à 900°C ainsi qu'une température de dégradation de 10% de la masse totale de 403°C (rampe de chauffe: 5°C/min). Le graphe d'analyse thermogravimétrique obtenu est fourni à la figure 2. Le produit réticulé obtenu était insoluble dans le dichlorométhane (le taux d'insoluble après 24h à température ambiante dans le dichlorométhane, puis séchage sous vide à 60°C pendant 24h a été mesuré à hauteur de 100 +/- 0,1%).

### Exemple 2 : synthèse d'un monomère polybenzoxazine à partir de furfurylaminométhylphénol et isophthalaldéhyde puis réticulation subséquente

On fait réagir la furfurylamine avec le salicylaldéhyde dans des proportions stoechiométriques dans le méthanol au reflux pendant 2h, dans le but de former l'imine correspondante. L'imine est réduite en amine avec 1 équivalent de NaBH₄ ajouté à 0°C dans une solution de l'imine dans le MeOH, puis on réalise un chauffage à reflux pendant 2 heures. Le furfurylaminométhylphénol ainsi synthétisé est dissous dans le toluène avec 0,5 équivalent d'isophthalaldéhyde puis porté au reflux dans un appareil Dean Stark, afin de retirer l'eau générée pendant la réaction de condensation. La réaction est arrêtée lorsque la conversion des aldéhydes a atteint son maximum, suivi par RMN du proton. Après évaporation du solvant sous pression réduite, la bisbenzoxazine isolée est un solide blanc cassé. Le produit a été caractérisé par RMN et la structure a été confirmée.

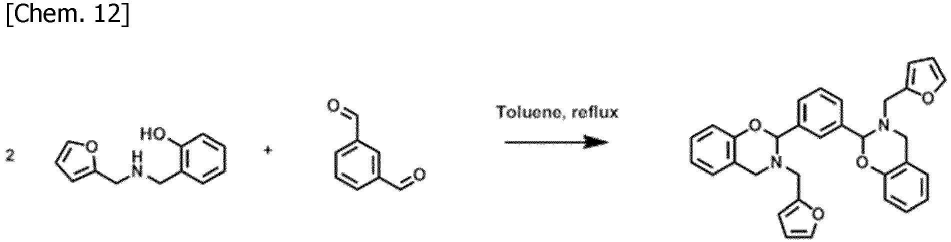

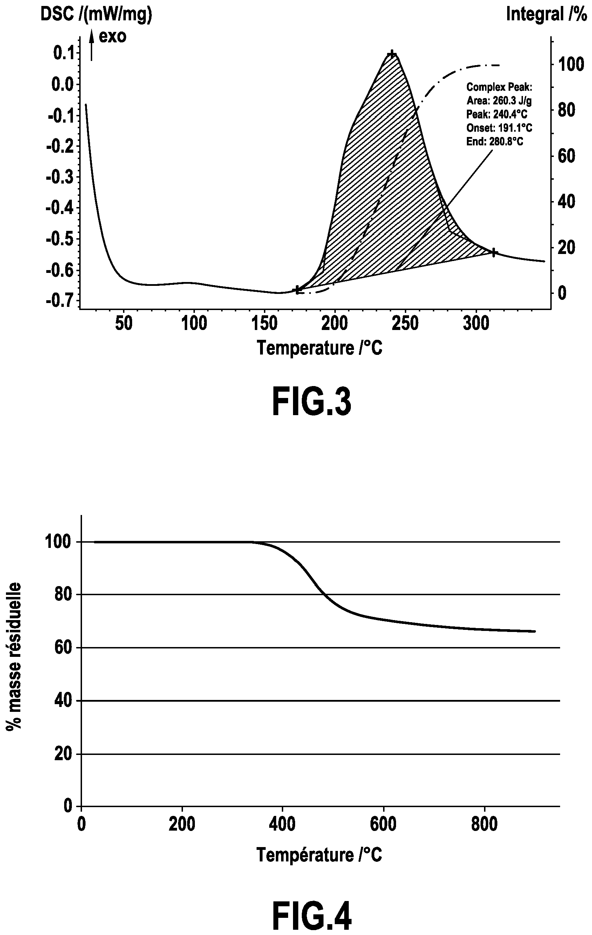

La caractérisation thermique par calorimétrie différentielle à balayage (« Differential Scanning Calorimetry » ; « DSC ») a révélé une réaction exothermique entre 190°C et 280°C, représentant 260J/g d'enthalpie par rapport à la référence, avec une rampe de 10°C/min dans des creusets en acier scellés haute pression. Le thermogramme DSC obtenu est fourni à la figure 3.

Un échantillon de bisbenzoxazine a été réticulé à 200°C pendant 10 heures et n'a pas montré de signal résiduel en DSC. L'analyse thermogravimétrique a montré un taux de coke de 66% sous atmosphère d'azote, après 1h à 900°C ainsi qu'une température de dégradation de 10% de la masse totale de 403°C (rampe de chauffe: 5°C/min). Le graphe d'analyse thermogravimétrique obtenu est fourni à la figure 4.

### Exemple 3 : synthèse d'un monomère polybenzoxazine à partir de benzylaminométhylphénol et téréphthalaldéhyde puis réticulation subséquente

On fait réagir la benzylamine avec le salicylaldéhyde dans des proportions stoechiométriques dans le méthanol au reflux pendant 2h, dans le but de former l'imine correspondante. L'imine est réduite en amine avec 1 équivalent de NaBH₄ ajouté à 0°C dans une solution de l'imine dans le MeOH, puis on réalise un chauffage à reflux pendant 2 heures. Le benzylaminométhylphénol ainsi synthétisé est dissous dans le toluène avec 0,5 équivalent de téréphthalaldéhyde puis porté au reflux dans un appareil Dean Stark, afin de retirer l'eau générée pendant la réaction de condensation. La réaction est arrêtée lorsque la conversion des aldéhydes a atteint son maximum, suivi par RMN du proton. Après évaporation du solvant sous pression réduite, la bisbenzoxazine isolée est un solide incolore. Le produit a été caractérisé par RMN et la structure a été confirmée.

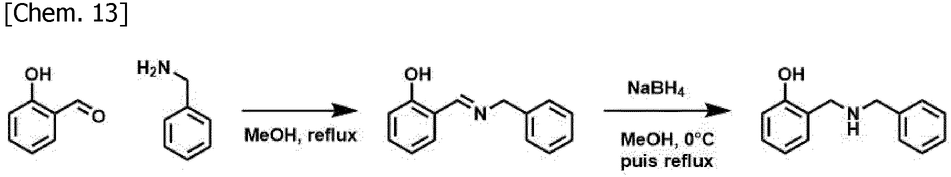

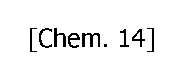

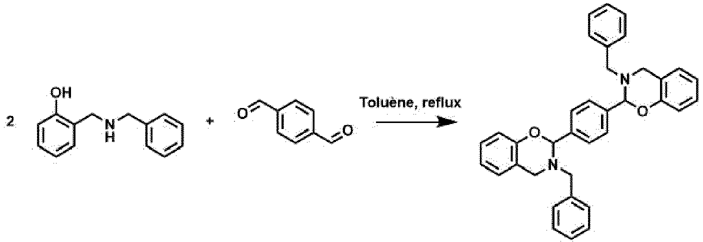

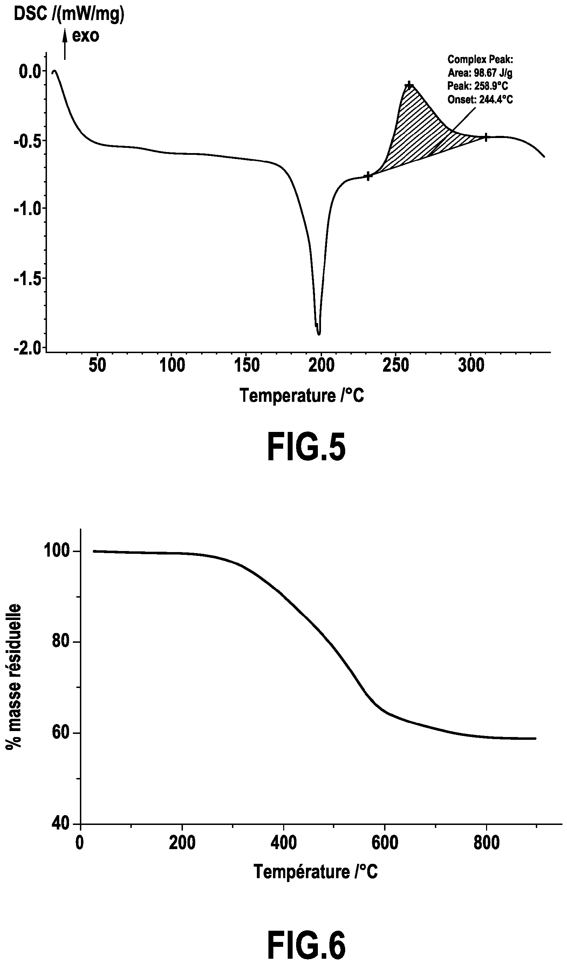

La caractérisation thermique par calorimétrie différentielle à balayage (« Differential Scanning Calorimetry » ; « DSC ») a révélé une réaction exothermique entre 240°C et 310°C, représentant 99J/g d'enthalpie par rapport à la référence, avec une rampe de 10°C/min dans des creusets en acier scellés haute pression. Le thermogramme DSC obtenu est fourni à la figure 5.

Un échantillon de bisbenzoxazine a été réticulé à 200°C pendant 7 heures et n'a pas montré de signal résiduel en DSC. L'analyse thermogravimétrique a montré un taux de coke de 59% sous atmosphère d'azote, ainsi qu'une température de dégradation de 10% de la masse totale de 401°C (rampe de chauffe: 10°C/min). Le graphe d'analyse thermogravimétrique obtenu est fourni à la figure 6.

### Exemple 4: étude d'un mélange d'un monomère polybenzoxazine avec un monomère additionnel monobenzoxazine

Un monomère additionnel monobenzoxazine a été synthétisé en suivant une procédure similaire à celle de l'exemple 1 à partir de benzaldéhyde et de furfurylaminométhylphénol avec des proportions 1 : 1 entre ces deux constituants. La température de fusion du monomère additionnel monobenzoxazine est de 52°C. La structure chimique du monomère additionnel monobenzoxazine obtenu est fournie ci-dessous.

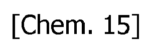

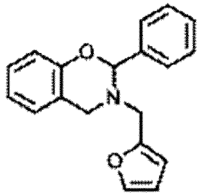

On a ensuite préparé des mélanges du monomère polybenzoxazine de l'exemple 1 et du monomère additionnel monobenzoxazine. Il a été constaté que la viscosité des mélanges obtenus était significativement inférieure à celle d'un milieu constitué uniquement du monomère polybenzoxazine. Les rapports massiques monobenzoxazine/polybenzoxazine des mélanges préparés étaient les suivants : 25/75 ; 50/50 et 65/35.

Ces mélanges de ces deux benzoxazines ont été réticulés à 200°C pendant 4h, résultant en un matériau totalement réticulé, insoluble dans le dichlorométhane (le taux d'insoluble après 24h à température ambiante dans le dichlorométhane, puis séchage sous vide à 60°C pendant 24h a été mesuré à hauteur de 100 +/- 0,1%).

Les résultats obtenus après analyse thermogravimétriques pour ces mélanges réticulés sont fournis dans le tableau 1 ci-dessous.

**[Table 1]**

| **Ratio mono/poly** | **Taux de coke à 900°C** | **Td 10%** | **Taux d'insolubles** |
|---|---|---|---|
| 0/100 | 62% | 403°C | >99,9% +/- 0,1% |
| 25/75 | 61% | 392°C | >99,9% +/- 0,1% |
| 50/50 | 58% | 381°C | >99,9%+/-0,1% |
| 65/35 | 56% | 375°C | >99,9% +/-0,1% |

L'analyse thermogravimétrique des mélanges monobenzoxazine/polybenzoxazine réalisés n'a montré qu'une faible baisse du taux de coke par rapport à l'emploi de la polybenzoxazine seule. La température de dégradation de 10% de la masse totale (T_{d}10%) n'est pas non plus significativement affectée. Ces mesures ont été conduites avec une rampe de 10°C/min jusqu'à 900°C, avec un isotherme d'1h à 900°C, sous atmosphère d'azote.

Le monomère monobenzoxazine additionnel synthétisé présente l'avantage de présenter un caractère liquide lorsqu'on le chauffe à température modérée. Son adjonction permet ainsi de faciliter l'obtention d'une phase liquide par rapport à l'emploi du monomère polybenzoxazine seul et ce sans significativement affecter les propriétés de stabilité thermique et de charbonnement.

### Exemple 5: étude d'un mélange d'un monomère polybenzoxazine synthétisé à partir d'un polyaldéhyde avec un monomère polybenzoxazine additionnel synthétisé à partir d'une polyamine

On a synthétisé un monomère polybenzoxazine additionnel à partir d'une polyamine de la manière suivante.

On fait réagir la méta-xylylènediamine avec le salicylaldéhyde dans des proportions stoechiométriques dans le méthanol au reflux pendant 2h, dans le but de former l'imine correspondante. L'imine est réduite en amine avec 2 équivalents de NaBH₄ ajouté à 0°C dans une solution de l'imine dans l'éthanol, on réalise ensuite un chauffage à reflux pendant 2 heures. Le méta-xylylène-aminométhylphénol ainsi synthétisé est dissous dans le toluène avec 2 équivalents de benzaldéhyde, puis porté au reflux dans un appareil Dean Stark, afin de retirer l'eau générée pendant la condensation. Après évaporation du solvant sous pression réduite, le produit obtenu est un liquide visqueux de couleur orangée à chaud, et solide jaune pâle à température ambiante. Le produit a été caractérisé par RMN et la structure a été confirmée.

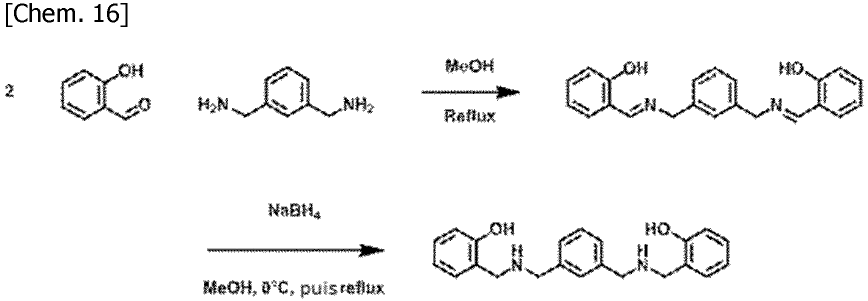

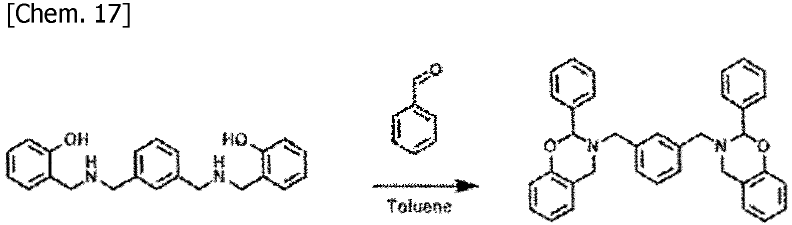

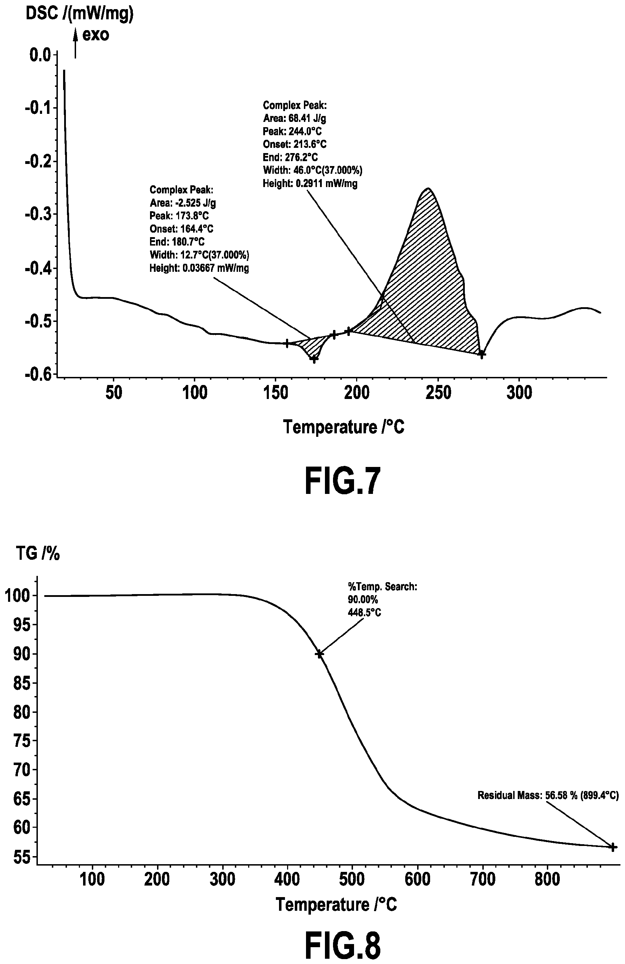

La caractérisation thermique par DSC a révélé une enthalpie de polymérisation de 68J/g. Le thermogramme DSC obtenu est fourni à la figure 7. L'analyse thermogravimétrique d'un échantillon réticulé a montré un taux de coke de 57% sous atmosphère d'azote à 900°C ainsi qu'une température de dégradation de 10% de la masse totale de 448°C. Le graphe d'analyse thermogravimétrique obtenu est fourni à la figure 8.

Le monomère polybenzoxazine additionnel synthétisé à partir la polyamine a été mélangé au monomère polybenzoxazine obtenu à l'exemple 1. Le monomère polybenzoxazine additionnel synthétisé à partir de la polyamine présente avantageusement un caractère liquide lorsqu'on le chauffe à température modérée. Son adjonction permet ainsi de faciliter l'obtention d'une phase liquide par rapport à l'emploi du monomère polybenzoxazine de l'exemple 1 seul.

On a réalisé un mélange comprenant le monomère polybenzoxazine obtenu à partir du polyaldéhyde à raison de 25% en masse et le monomère polybenzoxazine additionnel synthétisé à partir de la polyamine à raison de 75% en masse. Le mélange a été chauffé à 80°C à l'aide d'un bain marie, puis le mélange a été homogénéisé à la spatule et ensuite réticulé par traitement thermique à 200°C.

On a réalisé une analyse thermogravimétrique de ce mélange réticulé sous azote jusqu'à 900°C. Les résultats sont fournis au tableau 2 ci-dessous. On peut remarquer que le mélange de polybenzoxazines testé possède un taux de coke intermédiaire entre les produits réticulés à partir de polybenzoxazines pures. L'adjonction du monomère polybenzoxazine additionnel synthétisé à partir de la polyamine permet de diminuer significativement la viscosité, ce qui permet de faciliter le recours à certains types de techniques comme le procédé de moulage par transfert de résine. Dans le tableau 2 ci-dessous, le monomère polybenzoxazine additionnel synthétisé à partir de la polyamine est noté « bzx MXDA » et le monomère polybenzoxazine synthétisé à partir du polyaldéhyde est noté « bzx TPA ». On constate que les propriétés thermiques et de charbonnement du mélange demeurent compatibles de l'application en tant que résine ablative pour tuyère de propulseur.

**[Table 2]**

| **Ratio bzx MXDA/ bzx TPA** | **Taux de coke à 900°C** | **T_{d} 10%** |
|---|---|---|
| 100/0 | 57% | 448°C |
| 75 / 25 | 59% | 411°C |
| 0 / 100 | 62% | 405°C |

L'expression « compris(e) entre ... et ... » doit se comprendre comme incluant les bornes.

## Revendications

1. Procédé de fabrication d'un monomère polybenzoxazine comprenant la condensation d'une amine de formule A avec un polyaldéhyde de formule B afin d'obtenir le monomère polybenzoxazine de formule C, les formules A, B et C étant fournies ci-dessous : 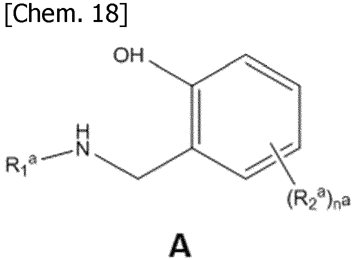 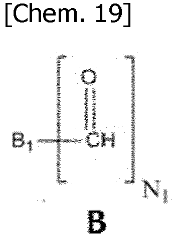 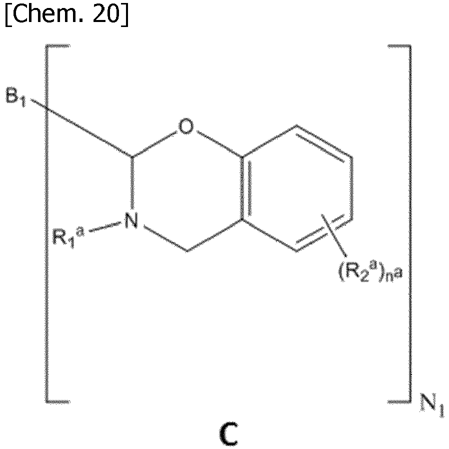 dans ces formules :
R₁^{a} est un groupe furfuryle substitué ou non substitué ou un groupe benzyle substitué ou non substitué,
R₂^{a} est choisi parmi : les groupes alcoxy, les groupes carboxyles, les atomes d'halogène, les chaînes hydrocarbonées linéaires ou ramifiées comprenant entre 1 et 6 atomes de carbone, saturées ou insaturées, substituées ou non substituées, interrompues ou non par un ou plusieurs hétéroatomes, les groupes carbocycliques ou hétérocycliques saturés, insaturés ou aromatiques, substitués ou non substitués,
n^{a} est un entier compris entre 0 et 2,
B₁ est choisi parmi : les groupes carbocycliques aromatiques ou hétérocycliques aromatiques, monocycliques ou polycycliques, substitués ou non substitués, et
N₁ est un entier supérieur ou égal à 2,
et, lorsqu'ils sont substitués, chacun de R₁^{a}, B₁ et R₂^{a} étant substitué par l'un au moins des groupements suivants : hydroxyméthyle, méthyle, acide carboxylique.

2. Procédé selon la revendication 1, dans lequel n^{a} est égal à 0.

3. Procédé selon la revendication 1 ou 2, dans lequel B₁ est un cycle benzénique substitué ou non substitué et dans ce cas N₁ = 2 ou 3.

4. Procédé selon l'une quelconque des revendications 1 à 3, dans lequel N₁ est un entier égal à 2.

5. Procédé de fabrication d'un produit réticulé comprenant :
- la fabrication d'un monomère polybenzoxazine de formule C par mise en oeuvre du procédé selon l'une quelconque des revendications 1 à 4, et
- la réticulation du monomère polybenzoxazine de formule C.

6. Procédé selon la revendication 5, dans lequel il y a réticulation d'un mélange comprenant le monomère polybenzoxazine de formule C et un monomère monobenzoxazine additionnel de formule D, la formule D étant fournie ci-dessous : 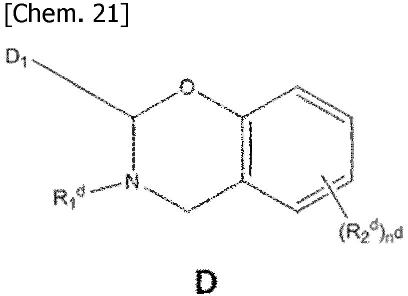 formule dans laquelle :
R₁^{d} est choisi parmi : les groupes furfuryles, substitués ou non substitués, les groupes carbocycliques ou hétérocycliques saturés, insaturés ou aromatiques, monocycliques ou polycycliques, substitués ou non substitués, les groupes aralkyles substitués ou non substitués, les chaînes hydrocarbonées linéaires ou ramifiées, saturées ou insaturées, substituées ou non substituées, interrompues ou non par un ou plusieurs hétéroatomes ou par un ou plusieurs groupes carbocycliques ou hétérocycliques saturés, insaturés ou aromatiques, monocycliques ou polycycliques, substitués ou non substitués,
R₂^{d} est choisi parmi : les groupes alcoxy, les groupes carboxyles, les atomes d'halogène, les chaînes hydrocarbonées linéaires ou ramifiées comprenant entre 1 et 6 atomes de carbone, saturées ou insaturées, substituées ou non substituées, interrompues ou non par un ou plusieurs hétéroatomes, les groupes carbocycliques ou hétérocycliques saturés, insaturés ou aromatiques, substitués ou non substitués,
n^{d} est un entier compris entre 0 et 2,
D₁ est choisi parmi : les groupes carbocycliques ou hétérocycliques saturés, insaturés ou aromatiques, monocycliques ou polycycliques, substitués ou non substitués, les chaînes hydrocarbonées linéaires ou ramifiées, saturées ou insaturées, substituées ou non substituées, interrompues ou non par un ou plusieurs hétéroatomes,
et, lorsqu'ils sont substitués, chacun de R₁^{d}, R₂^{d} et D₁ étant substitué par l'un au moins des groupements suivants : hydroxyméthyle, méthyle, acide carboxylique.

7. Procédé selon la revendication 5, dans lequel il y a réticulation d'un mélange comprenant le monomère polybenzoxazine de formule C et un monomère polybenzoxazine additionnel de formule E, la formule E étant fournie ci-dessous : 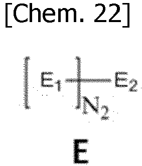 dans cette formule E :
N₂ est un entier supérieur ou égal à 2,
E₂ est choisi parmi les chaînes hydrocarbonées linéaires ou ramifiées saturées ou insaturées, substituées ou non substituées, interrompues par un ou plusieurs hétéroatomes ou par un ou plusieurs groupes carbocycliques ou hétérocycliques saturés, insaturés ou aromatiques, monocycliques ou polycycliques, substitués ou non substitués, ou non interrompues,
les groupements de formule E₁ sont identiques ou différents et présentent chacun la formule ci-dessous : 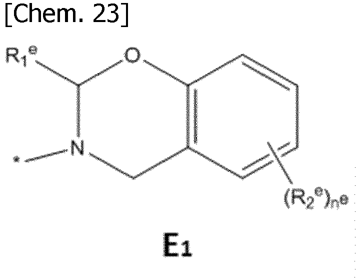 dans cette formule E₁ :
R₁^{e} est choisi parmi : les groupes carbocycliques ou hétérocycliques saturés, insaturés ou aromatiques, monocycliques ou polycycliques, substitués ou non substitués, les chaînes hydrocarbonées linéaires ou ramifiées, saturées ou insaturées, substituées ou non substituées, interrompues ou non par un ou plusieurs hétéroatomes, et
R₂^{e} est choisi parmi : les groupes alcoxy, les groupes carboxyles, les atomes d'halogène, les chaînes hydrocarbonées linéaires ou ramifiées comprenant entre 1 et 6 atomes de carbone, saturées ou insaturées, substituées ou non substituées, interrompues ou non par un ou plusieurs hétéroatomes, les groupes carbocycliques ou hétérocycliques saturés, insaturés ou aromatiques, substitués ou non substitués,
n^{e} est un entier compris entre 0 et 2,
*- désigne la liaison à E₂,
et, lorsqu'ils sont substitués, chacun de E₂, R₁^{e} et R₂^{e} étant substitué par l'un au moins des groupements suivants : hydroxyméthyle, méthyle, acide carboxylique.

8. Procédé de fabrication d'une tuyère de propulseur comprenant la fabrication du produit réticulé par mise en oeuvre du procédé selon l'une quelconque des revendications 5 à 7, et la fabrication de la tuyère en mettant en oeuvre ledit produit réticulé.

9. Procédé de fabrication d'un corps de rentrée atmosphérique comprenant la fabrication du produit réticulé par mise en oeuvre du procédé selon l'une quelconque des revendications 5 à 7, et la fabrication du corps de rentrée atmosphérique en mettant en oeuvre ledit produit réticulé.

## Patentansprüche

1. Verfahren zur Herstellung eines Polybenzoxazin-Monomers, umfassend die Kondensation eines Amins der Formel A mit einem Polyaldehyd der Formel B, um das Polybenzoxazin-Monomer der Formel C zu erhalten, wobei die Formeln A, B und C nachfolgend bereitgestellt sind: 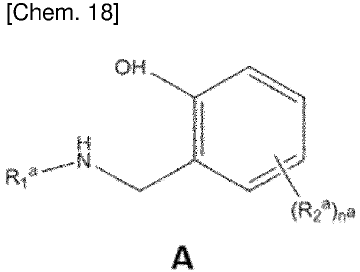 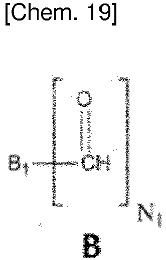 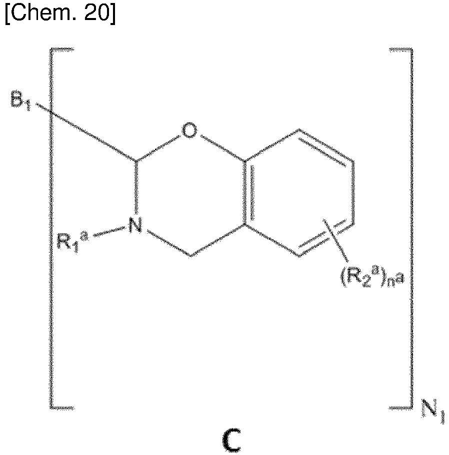 wobei in diesen Formeln:
R₁^{a} eine substituierte oder nicht substituierte Furfurylgruppe oder eine substituierte oder nicht substituierte Benzylgruppe ist,
R₂^{a} ausgewählt ist aus: Alkoxygruppen, Carboxylgruppen, Halogenatomen, linearen oder verzweigten Kohlenwasserstoffketten, die zwischen 1 und 6 Kohlenstoffatomen umfassen, gesättigt oder ungesättigt, substituiert oder nicht substituiert, unterbrochen oder nicht durch ein oder mehrere Heteroatome, carbocyclischen oder
heterocyclischen gesättigten, ungesättigten oder aromatischen, substituierten oder nicht substituierten Gruppen,
n^{a} eine ganze Zahl zwischen 0 und 2 ist,
B₁ ausgewählt ist aus: carbocyclischen aromatischen oder heterocyclischen aromatischen, monocyclischen oder polycyclischen, substituierten oder nicht substituierten Gruppen, und
N₁ eine ganze Zahl größer als oder gleich 2 ist,
und, wenn sie substituiert sind, jedes von R₁^{a}, B₁ und R₂^{a} durch mindestens eine der folgenden Gruppierungen substituiert ist: Hydroxymethyl, Methyl, Carbonsäure.

2. Verfahren nach Anspruch 1, wobei n^{a} gleich 0 ist.

3. Verfahren nach Anspruch 1 oder 2, wobei B₁ ein substituierter oder nicht substituierter Benzolring ist und in diesem Fall N₁ = 2 oder 3.

4. Verfahren nach einem der Ansprüche 1 bis 3, wobei N₁ eine ganze Zahl gleich 2 ist.

5. Verfahren zur Herstellung eines vernetzten Produkts, umfassend:
- Herstellung eines Polybenzoxazin-Monomers der Formel C durch Umsetzung des Verfahrens nach einem der Ansprüche 1 bis 4, und
- Vernetzung des Polybenzoxazin-Monomers der Formel C.

6. Verfahren nach Anspruch 5, wobei es eine Vernetzung einer Mischung gibt, die das Polybenzoxazin-Monomer der Formel C und ein zusätzliches Monobenzoxazin-Monomer der Formel D umfasst, wobei die Formel D nachfolgend bereitgestellt ist: 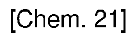 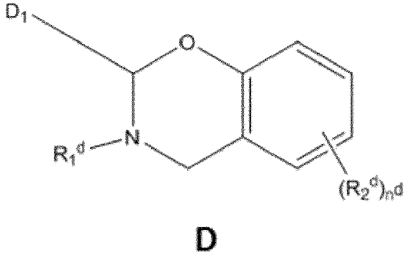 wobei in der Formel:
R₁^{d} ausgewählt ist aus: substituierten oder nicht substituierten Furfurylgruppen, carbocyclischen oder heterocyclischen gesättigten, ungesättigten oder aromatischen, monocyclischen oder polycyclischen, substituierten oder nicht substituierten Gruppen, substituierten oder nicht substituierten Aralkylgruppen, linearen oder verzweigten gesättigten oder ungesättigten, substituierten oder nicht substituierten Kohlenwasserstoffketten, unterbrochen oder nicht durch ein oder mehrere Heteroatome oder durch eine oder mehrere carbocyclische oder heterocyclische gesättigte, ungesättigte oder aromatische, monocyclische oder polycyclische, substituierte oder nicht substituierte Gruppen,
R₂^{d} ausgewählt ist aus: Alkoxygruppen, Carboxylgruppen, Halogenatomen, linearen oder verzweigten Kohlenwasserstoffketten, die zwischen 1 und 6 Kohlenstoffatomen umfassen, gesättigt oder ungesättigt, substituiert oder nicht substituiert, unterbrochen oder nicht durch ein oder mehrere Heteroatome, carbocyclischen oder heterocyclischen gesättigten, ungesättigten oder aromatischen, substituierten oder nicht substituierten Gruppen,
n^{d} eine ganze Zahl zwischen 0 und 2 ist,
D₁ ausgewählt ist aus: carbocyclischen oder heterocyclischen gesättigten,
ungesättigten oder aromatischen, monocyclischen oder polycyclischen, substituierten oder nicht substituierten Gruppen, linearen oder verzweigten, gesättigten oder ungesättigten, substituierten oder nicht substituierten Kohlenwasserstoffketten, unterbrochen oder nicht durch ein oder mehrere Heteroatome, und, wenn sie substituiert sind, jedes von R₁^{d}, R₂^{d} und D₁ durch mindestens eine der folgenden Gruppierungen substituiert ist: Hydroxymethyl, Methyl, Carbonsäure.

7. Verfahren nach Anspruch 5, wobei es eine Vernetzung einer Mischung gibt, die das Polybenzoxazin-Monomer der Formel C und ein zusätzliches Polybenzoxazin-Monomer der Formel E umfasst, wobei die Formel E nachfolgend bereitgestellt ist: 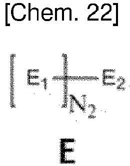 wobei in dieser Formel E:
N₂ eine ganze Zahl größer als oder gleich 2 ist,
E₂ ausgewählt ist aus linearen oder verzweigten gesättigten oder ungesättigten, substituierten oder nicht substituierten Kohlenwasserstoffketten, unterbrochen oder nicht durch ein oder mehrere Heteroatome oder durch eine oder mehrere carbocyclische oder heterocyclische gesättigte, ungesättigte oder aromatische, monocyclische oder polycyclische, substituierte oder nicht substituierte Gruppen, oder nicht unterbrochen,
wobei die Gruppen der Formel E₁ identisch oder verschieden sind und jeweils die nachfolgende Formel aufweisen: 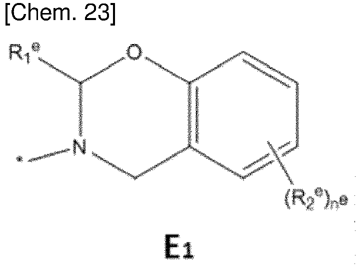
wobei in dieser Formel E₁:
R₁^{e} ausgewählt ist aus: carbocyclischen oder heterocyclischen gesättigten,
ungesättigten oder aromatischen, monocyclischen oder polycyclischen, substituierten oder nicht substituierten Gruppen, linearen oder verzweigten, gesättigten oder ungesättigten, substituierten oder nicht substituierten Kohlenwasserstoffketten, unterbrochen oder nicht durch ein oder mehrere Heteroatome, und
R₂^{e} ausgewählt ist aus: Alkoxygruppen, Carboxylgruppen, Halogenatomen, linearen oder verzweigten Kohlenwasserstoffketten, die zwischen 1 und 6 Kohlenstoffatomen umfassen, gesättigt oder ungesättigt, substituiert oder nicht substituiert, unterbrochen oder nicht durch ein oder mehrere Heteroatome, carbocyclischen oder
heterocyclischen gesättigten, ungesättigten oder aromatischen, substituierten oder nicht substituierten Gruppen,
n^{e} eine ganze Zahl zwischen 0 und 2 ist,
*- die Verbindung mit E₂ bezeichnet,
und, wenn sie substituiert sind, jedes von E₂, R₁^{e} und R₂^{e} durch mindestens eine der folgenden Gruppierungen substituiert ist: Hydroxymethyl, Methyl, Carbonsäure.

8. Verfahren zur Herstellung einer Triebwerksdüse, umfassend die Herstellung des vernetzten Produkts durch Umsetzung des Verfahrens nach einem der Ansprüche 5 bis 7 und die Herstellung der Düse durch Umsetzung des vernetzten Produkts.

9. Verfahren zur Herstellung eines Atmosphärenwiedereintrittskörpers, umfassend die Herstellung des vernetzten Produkts durch Umsetzung des Verfahrens nach einem der Ansprüche 5 bis 7 und die Herstellung des Atmosphärenwiedereintrittskörpers durch Umsetzung des vernetzten Produkts.

## Claims

1. A process for manufacturing a polybenzoxazine monomer comprising condensing an amine of formula A with a polyaldehyde of formula B in order to obtain the polybenzoxazine monomer of formula C, formulas A, B and C being provided below: 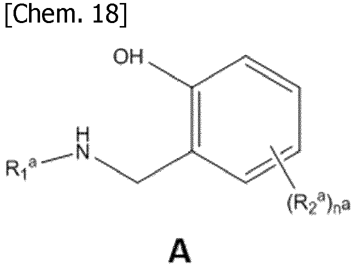 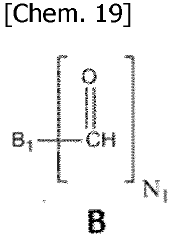 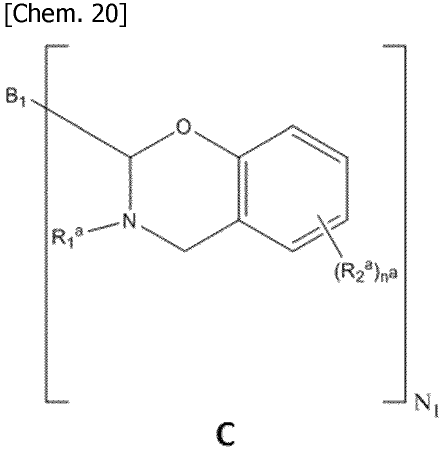 in these formulas:
R₁^{a} is a substituted or unsubstituted furfuryl group or a substituted or unsubstituted benzyl group,
R₂^{a} is selected from: alkoxy groups; carboxyl groups; halogen atoms; saturated or unsaturated, substituted or unsubstituted, linear or branched hydrocarbon chains comprising between 1 and 6 carbon atoms, optionally interrupted by one or more heteroatoms; saturated, unsaturated or aromatic, substituted or unsubstituted carbocyclic or heterocyclic groups;
n^{a} is an integer comprised between 0 and 2;
B₁ is selected from: aromatic, monocyclic or polycyclic, substituted or unsubstituted carbocyclic or heterocyclic groups; and
N₁ is an integer greater than or equal to 2,
and, when substituted, each of R₁^{a}, B₁ and R₂^{a} being substituted with at least one of the following groups: hydroxymethyl, methyl, carboxylic acid.

2. The process as claimed in claim 1, wherein n^{a} is equal to 0.

3. The process as claimed in claim 1 or 2, wherein B₁ is a substituted or unsubstituted benzene ring and, in this case, N₁ = 2 or 3.

4. The process as claimed in any one of claims 1 to 3, wherein N₁ is an integer equal to 2.

5. A process for manufacturing a crosslinked product comprising:
- manufacturing a polybenzoxazine monomer of formula C by carrying out the process as claimed in any one of claims 1 to 4, and
- crosslinking the polybenzoxazine monomer of formula C.

6. The process as claimed in claim 5, wherein there is crosslinking of a mixture comprising the polybenzoxazine monomer of formula C and an additional monobenzoxazine monomer of formula D, formula D being provided below: 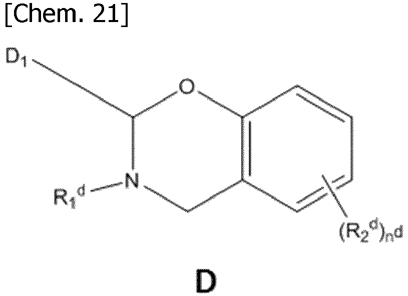 formula in which:
R₁^{d} is selected from: substituted or unsubstituted furfuryl groups; saturated, unsaturated or aromatic, monocyclic or polycyclic, substituted or unsubstituted carbocyclic or heterocyclic groups; substituted or unsubstituted aralkyl groups;
saturated or unsaturated, substituted or unsubstituted, linear or branched hydrocarbon chains , optionally interrupted by one or more heteroatoms or by one or more saturated, unsaturated or aromatic, monocyclic or polycyclic, substituted or unsubstituted carbocyclic or heterocyclic groups;
R₂^{d} is selected from: alkoxy groups; carboxyl groups; halogen atoms; saturated or unsaturated, substituted or unsubstituted, linear or branched hydrocarbon chains comprising between 1 and 6 carbon atoms, optionally interrupted by one or more heteroatoms; saturated, unsaturated or aromatic, substituted or unsubstituted carbocyclic or heterocyclic groups;
n^{d} is an integer comprised between 0 and 2;
D₁ is selected from: saturated, unsaturated or aromatic, monocyclic or polycyclic, substituted or unsubstituted carbocyclic or heterocyclic groups; linear or branched, saturated or unsaturated, substituted or unsubstituted hydrocarbon chains,
optionally interrupted by one or more heteroatoms,
and, when substituted, each of R₁^{d}, R₂^{d} and D₁ being substituted with at least one of the following groups: hydroxymethyl, methyl, carboxylic acid.

7. The process as claimed in claim 5, wherein there is crosslinking of a mixture comprising the polybenzoxazine monomer of formula C and an additional polybenzoxazine monomer of formula E, formula E being provided below: 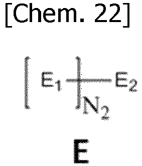 in this formula E:
N₂ is an integer greater than or equal to 2;
E₂ is selected from saturated or unsaturated, substituted or unsubstituted, linear or branched hydrocarbon chains interrupted by one or more heteroatoms or by one or
more saturated, unsaturated or aromatic, monocyclic or polycyclic, substituted or unsubstituted, carbocyclic or heterocyclic groups, or uninterrupted;
the groups of formula E₁ are identical or different and each has the formula below: 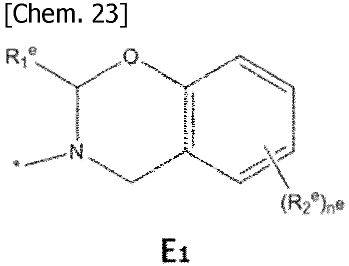 in this formula E₁:
R₁^{e} is selected from: saturated, unsaturated or aromatic, monocyclic or polycyclic, substituted or unsubstituted carbocyclic or heterocyclic groups; linear or branched, saturated or unsaturated, substituted or unsubstituted hydrocarbon chains,
optionally interrupted by one or more heteroatoms; and
R₂^{e} is selected from: alkoxy groups; carboxyl groups; halogen atoms; saturated or unsaturated, substituted or unsubstituted, linear or branched hydrocarbon chains comprising between 1 and 6 carbon atoms, optionally interrupted by one or more heteroatoms; saturated, unsaturated or aromatic, substituted or unsubstituted carbocyclic or heterocyclic groups;
n^{e} is an integer comprised between 0 and 2;
*- denotes the bond to E₂,
and, when substituted, each of E₂, R₁^{e}, R₂^{e} being substituted with at least one of the following groups: hydroxymethyl, methyl, carboxylic acid.

8. A process for manufacturing a thruster nozzle comprising manufacturing the crosslinked product by carrying out the process as claimed in any one of claims 5 to 7 and manufacturing the nozzle by using said crosslinked product.

9. A process for manufacturing an atmospheric reentry body comprising manufacturing the crosslinked product by carrying out the process as claimed in any one of claims 5 to 7 and manufacturing the atmospheric reentry body by using said crosslinked product.
